# EUROPÄISCHE PATENTANMELDUNG

(11) **EP 0 526 710 A2**
(43) Veröffentlichungstag der Anmeldung: **10.02.1993**
(21) Anmeldenummer: 92109621.0
(22) Anmeldetag: 06.06.1992
(51) Int. Cl.: C07H 15/04

(54) **Verfahren zur Bleichung von Alkylpolyglycosiden**

(30) Priorität: 06.08.1991 DE 4125979
(71) Anmelder: HÜLS AKTIENGESELLSCHAFT, D-45764 Marl (DE)
(72) Erfinder: Ripke, Norbert, Dr., W-4358 Haltern (DE)

(57) **Zusammenfassung**

Nach dem vorliegenden Verfahren werden 10- bis 80%ige Lösungen von Alkylpolyglycosiden durch Bestrahlung mit 100- bis 1 000-W-Lampen gebleicht.

## Beschreibung

Die Erfindung betrifft ein neues Verfahren zur Herstellung von Alkylpolyglycosiden mit verbesserter Farbqualität.

Alkylpolyglycoside sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 C-Atome aufweisen.

Alkylpolyglycoside mit langkettigen Alkylgruppen werden im allgemeinen durch ein- oder mehrstufige Synthesen hergestellt.

Ein einstufiges Herstellverfahren wird u. a. in der deutschen Patentanmeldung mit dem Aktenzeichen P 41 01 252.6 beschrieben.

Ein zweistufiges Verfahren wird beispielsweise in EP-A-0 306 652 angegeben, wonach man zunächst durch Glycosidierung mit n-Butanol ein n-Butylglycosid und daraus durch Umglycosidierung mit einem langkettigen Alkohol das gewünschte langkettige Alkylpolyglycosid herstellt.

Für Anwendungen im Haushalt und im Kosmetikbereich müssen diese Produkte hohen ästhetischen Ansprüchen genügen. Man ist daher allgemein an Verfahren interessiert, nach denen Alkylpolyglycoside in transparenten, farblich schönen wäßrigen Lösungen hergestellt werden können.

Die Farbe kann nach US 4 762 918 durch katalytische Hydrierung verbessert werden.

Bei der Herstellung von langkettigen Alkylsacchariden können nach US 4 465 828 auch die Hydroxylpolycarbonsäuren Citronensäure, Weinsäure und Äpfelsäure zur Farbverbesserung beitragen.

Nach EP 0 077 167 können bei der Umsetzung von Alkoholen mit Aldosen oder Ketosen Reduktionsmittel, wie hypophosphorige Säure oder schwefelige Säure, zugesetzt werden. Dadurch wird die Farbe der Alkylglycoside verbessert.

Es sind auch vorbeugende Maßnahmen bekannt. So erhält man nach EP 0 102 558 farblich verbesserte C₃- bis C₅-Alkylglucoside, wenn man die Glucosidierung in Gegenwart eines Alkalisalzes einer Borsäure durchführt.

Nach EP 0 165 721 kann die Farbe der Produkte durch mehrstufige Bleichung mit Wasserstoffperoxid verbessert und durch Zusatz von Schwefeldioxid freisetzenden Verbindungen stabilisiert werden.

Die katalytische Hydrierung ist wegen der hohen Katalysatorkosten und der schwierigen Reaktionsführung problematisch. Die Wasserstoffperoxid-Bleichung erfordert bei großtechnischen Verfahren die Lagerung und Handhabung großer Mengen an Peroxid. Bei allen festen und flüssigen Farbverbesserern ist es außerdem schwierig, diese Mittel nach beendeter Reaktion quantitativ zu entfernen.

Es bestand daher die Aufgabe, ein in Verfahrensweise und Handhabung vereinfachtes Verfahren zur Farbverbesserung von Alkylpolyglycosiden bereitzustellen und dabei die Nachteile des Standes der Technik zu vermeiden.

Die Aufgabe wird erfindungsgemäß dadurch gelöst, daß man eine 10- bis 80%ige Lösung von Alkylpolyglycosiden mit einer 100- bis 1 000-W-Lampe bestrahlt.

Die hier eingesetzten Alkylpolyglycoside können nach allen bekannten Methoden, beispielsweise einstufig oder zweistufig durch Glycosidierung und Umglycosidierung, hergestellt werden. Sie enthalten Alkylgruppen mit 8 bis 24 C-Atomen, wobei Alkylgruppen mit 10 bis 18 C-Atomen vorzugsweise vorhanden sind. Der mittlere Polymerisationsgrad liegt bei 1 bis 10, vorzugsweise bei 1,1 bis 3. Die Glycosideinheiten können dabei beispielsweise von Glucose, Mannose, Maltose oder Lactose hergeleitet werden.

Geeignete Lösemittel sind Wasser und polare organische Lösemittel, wie beispielsweise Methanol, Ethanol, i-Propanol, n-Butanol, Hexanol, Methylenchlorid, Dichlormethan, Diethylether,Tetrahydrofuran, Aceton oder Methylethylketon. Vorzugsweise verwendet man Wasser, wobei man im besonderen 40- bis 70%ige wäßrige Alkylpolyglycosid-Lösungen zur Bleichung einsetzt.

Bei wäßrigen Lösungen liegt der pH-Wert meist bei 2 bis 11.

Während der Bestrahlung liegt die Temperatur der Lösung im allgemeinen bei 10 bis 90 °C. Dabei werden produktschonende Temperaturen von 15 bis 40 °C vorzugsweise eingestellt.

Die Bestrahlung kann mit Hilfe von handelsüblichen Glühbirnen durchgeführt werden. Einsetzbar sind beispielsweise auch Edelgas-, Quecksilberdampf- und Halogenlampen. Geeignet sind zum Beispiel Lampen von Dia-Projektoren und Quecksilber-Mitteldrucklampen. Vorzugsweise verwendet man 100- und 250-W-Lampen.

Bei Lampen mit < 100 W dauert die Bleichung unwirtschaftlich lange. Auf der anderen Seite sind Lampen mit > 1 000 W zwar auch geeignet, die Wärmeentwicklung und -abfuhr dieser Lampen ist jedoch etwas problematisch.

Die Bestrahlung kann in normalen Rührkesseln oder anderen üblichen Reaktoren und Gefäßen durchgeführt werden. Vorteilhaft sind spezielle Bestrahlungsapparaturen, die zur Erhöhung der Quantenausbeute von außen verspiegelt sind und die zur Ableitung der Wärme gekühlt werden können.

Die mittlere Verweilzeit der Alkylpolyglycosid-Lösung in der Bestrahlungsapparatur richtet sich nach der gewünschten Farbverbesserung. Sie ist auch abhängig von der Stärke der Lampe und von der Temperatur. Im allgemeinen liegt die mittlere Verweilzeit bei 2 Minuten bis 2 Stunden, wobei mittlere Verweilzeiten von 10 Minuten bis 1 Stunde bevorzugt werden.

Nach dem vorliegenden Verfahren kann die Farbe von Alkylpolyglycosiden bei geringem Aufwand drastisch verbessert werden.

Das Verfahren kann kontinuierlich durchgeführt werden. Ein nachfolgender Reinigungsschritt kann hier entfallen, da keine überschüssigen Bleichmittel abgetrennt werden müssen. Die durch Bestrahlung gebleichten Alkylpolyglycoside können direkt für Wasch- und Reinigungsmittel verwendet werden. Im besonderen können diese Alkylpolyglycoside auch als Emulgatoren in pharmazeutischen Produkten und Kosmetikformulierungen eingesetzt werden.

Das folgende Beispiel soll die Erfindung verdeutlichen.

### Beispiel

Durch eine Bleichapparatur mit einem Volumen von 175 ml wird eine 50%ige wäßrige Alkylpolyglycosid-Lösung (Alkyl = Dodecyl, Polymerisationsgrad 1,4) durchgeleitet. Die Lösung wird bei einer mittleren Verweilzeit von 25 Minuten mit einer 125-W-Quecksilbermitteldrucklampe bestrahlt. Die Produkttemperatur übersteigt dabei nicht 30 °C. Aus der zuvor dunkelbraun gefärbten Lösung mit einer Iodfarbzahl (IFZ) von > 500 wird durch die Bleichung eine Lösung mit einer IFZ von 20 bis 30 erhalten.

## Patentansprüche

1. Verfahren zur Bleichung von Alkylpolyglycosiden,
dadurch gekennzeichnet,
daß man eine 10- bis 80%ige Lösung von Alkylpolyglycosiden mit einer 100- bis 1 000-W-Lampe bestrahlt.

2. Verfahren nach Anspruch 1,
dadurch gekennzeichnet,
daß man eine 40- bis 70%ige wäßrige Lösung von Alkylpolyglycosiden bestrahlt.

3. Verfahren nach Anspruch 1,
dadurch gekennzeichnet,
daß man die Lösung mit einer 100- bis 250-W-Lampe bestrahlt.
